# FASCICULE DE BREVET EUROPEEN

(11) **EP 1 718 254 B1**
(45) Date de publication et mention de la délivrance du brevet: **28.05.2008**
(21) Numéro de dépôt: 05729310.2
(22) Date de dépôt: 18.02.2005
(51) Int. Cl.: A61F 5/00

(54) **ANNEAU CHIRURGICAL ATRAUMATIQUE**
ATRAUMATISCHES CHIRURGISCHES BAND
ATRAUMATIC SURGICAL BAND

(30) Priorité: 19.02.2004 FR 0401684
(43) Date de publication de la demande: 08.11.2006
(73) Titulaire: Compagnie Européenne d'Etude et de Recherche de Dispositifs pour l'Implantation par Laparoscopie, 38200 Vienne (FR)
(72) Inventeur: PAGANON, Pascal, F-69360 Serezin du Rhône (FR); RICOL, Jean-Paul, Gilbert, 69210 Saint Germain sur L'Arbresle (FR)
(74) Mandataire: Martin, Didier Roland Valéry
(86) Numéro de dépôt international: PCT/FR2005/000387
(87) Numéro de publication internationale: WO 2005/089684

(56) Documents cités:
- EP-A- 1 342 458
- WO-A-03/059215
- FR-A- 2 834 631

## Description

### DOMAINE TECHNIQUE

La présente invention se rapporte au domaine technique général des implants chirurgicaux destinés à être implantés dans le corps d'un patient autour d'organe(s) biologique(s) constituant une poche ou un conduit, et plus - particulièrement aux anneaux gastriques conçus pour traiter l'obésité par implantation d'un anneau gastrique souple, destiné à former une boucle fermé autour de l'estomac pour réduire le diamètre de l'ouverture du stoma.

La présente invention concerne un anneau chirurgical destiné à être implanté dans le corps d'un patient autour d'organe(s) biologique(s) constituant une poche ou un conduit pour modifier la section de passage dudit organe lorsqu'il est enserré par l'anneau, ledit anneau étant formé par une bande souple destinée à être fermée sensiblement vers ses deux extrémités pour former une boucle close, ladite bande comportant une chambre de compression annulaire destinée à contenir un fluide de remplissage, ladite chambre étant délimitée d'une part par une paroi interne destinée à être en contact avec l'organe à enserrer, et d'autre part par une paroi dorsale.

La présente invention concerne plus particulièrement un anneau de gastroplastie, mais elle vise aussi un anneau conçu pour être utilisé pour traiter l'incontinence urinaire ou fécale (sphincter artificiel), ou encore un anneau conçu pour régler le débit sanguin dans des vaisseaux sanguins, cette liste n'étant nullement limitative.

La présente invention concerne également un procédé de fabrication d'un anneau chirurgical destiné à être implanté dans le corps d'un patient autour d'organe(s) biologique(s) constituant une poche ou un conduit pour modifier la section de passage dudit organe lorsqu'il est enserré par l'anneau, dans lequel on réalise une chambre de compression annulaire destinée à contenir un fluide de remplissage, ladite chambre étant délimitée d'une part par une paroi interne destinée à être en contact avec l'organe à enserrer et d'autre part par une paroi dorsale.

### TECHNIQUE ANTERIEURE

Il est déjà connu d'intervenir de manière chirurgicale sur des patients atteints d'obésité extrêmement sévère (obésité morbide), c'est-à-dire dans le cas de patients dont le poids excède par exemple le poids idéal d'au moins 50 Kg, en implantant des anneaux de gastroplastie sur de tels patients. De telles interventions permettent d'éviter non seulement une série de problèmes de santé graves provenant d'un tel surpoids, mais encore et surtout d'éviter une mort certaine et proche de ces patients.

Il est en effet acquis que les patients souffrant d'obésité morbide voient leur espérance de vie réduite de manière importante, et d'au moins une dizaine à une quinzaine d'années, tout en souffrant d'importants problèmes de charge psychologique.

Par ailleurs, toute une série de phénomènes annexes de santé sont impliqués, ayant une incidence sur l'apparition de maladies annexes, telles que des maladies cardiovasculaires, l'hypertension, le diabète, ou encore des arthrites sévères notamment.

Il est également acquis que, pour de tels patients, les traitements basés sur des diètes sévères combinées à une série d'exercices physiques, associés également à une modification du comportement, notamment alimentaire, sont généralement peu adaptés, même si ces méthodes de traitement sont reconnues comme étant les plus saines.

C'est la raison pour laquelle les traitements efficaces et à long terme de l'obésité morbide font intervenir un traitement chirurgical.

De manière générale, on distingue des techniques de traitement chirurgical faisant intervenir un défaut d'absorption des aliments, c'est-à-dire un raccourcissement du passage des aliments et des sucs digestifs, et des techniques faisant intervenir une restriction gastrique réduisant la taille de l'estomac.

Les techniques chirurgicales impliquant un défaut d'absorption sont celles impliquant par exemple une technique de « *by pass* » ou de dérivation du petit intestin, ou encore celles mettant en oeuvre une séparation du passage des aliments relativement aux sucs-digestifs.

Ces techniques sont désormais rarement utilisées, car elles peuvent donner lieu à de sévères complications pour le patient et nécessitent dans tous les cas une importante intervention chirurgicale.

C'est la raison pour laquelle on tend désormais à privilégier les techniques chirurgicales mettant en oeuvre une restriction gastrique pour réduire la prise d'aliments.

Ces techniques largement connues font intervenir l'utilisation d'anneaux de gastroplastie implantés autour de l'estomac du patient, en vue de réduire sa taille ainsi que le diamètre de son passage (stoma).

La structure générale des anneaux de gastroplastie utilisés est bien connue et fait intervenir une bande souple, réalisée en matériau élastomère, destinée à être fermée vers deux extrémités par des moyens de fermeture autour de l'estomac d'un patient, pour réduire le diamètre de l'ouverture du stoma. Les moyens de fermeture sont généralement situés sur la partie externe ou dorsale de la bande souple, et font intervenir différents types de verrouillage, par exemple un verrouillage mécanique avec ou sans suturage.

Les anneaux connus comportent également une bande avec une chambre de compression annulaire dont le volume ou l'expansion diamétrale est réglable, ladite chambre étant susceptible d'être reliée par un cathéter à un dispositif de réglage du diamètre de la chambre par injection ou retrait de fluide. Grâce à cette particularité, on peut ainsi, à partir d'un anneau de taille ou de diamètre fixe, régler finement le diamètre interne de l'anneau par injection ou retrait de fluide, ce qui provoque une expansion ou une rétraction diamétrale correspondante de l'anneau.

Les dispositifs connus du type mentionné ci-dessus donnent généralement satisfaction mais souffrent d'un certain nombre de problèmes, et en particulier de problèmes de tolérance par le patient.

Il s'avère en effet particulièrement important de réduire autant que possible la sensation de gène procurée par de tels anneaux au niveau de la zone de restriction de l'estomac et d'éviter ou de réduire l'apparition de lésions tissulaires dans la zone de restriction.

A cette fin, il est nécessaire que la surface de l'anneau destinée à être en contact avec les tissus biologiques de l'estomac soit réalisée en un matériau particulièrement doux au contact, et que cette surface présente également un caractère lisse et régulier, et soit exempte en particulier de plis.

Afin de répondre notamment à cette contrainte d'atraumatisme, sans sacrifier par ailleurs la robustesse de l'anneau, il a été proposé par la demanderesse un anneau de gastroplastie muni d'un renfort dorsal réalisé à partir d'un premier matériau élastomère dont la dureté est supérieure à celle d'un deuxième matériau élastomère à partir duquel sont réalisées les parois latérales de la chambre de compression annulaire, et en particulier les parois destinées à entrer en contact avec les tissus de l'estomac.

Cet anneau, s'il s'avère effectivement présenter un excellent compromis entre l'atraumatisme, la robustesse et la simplicité de réalisation, n'en reste pas moins relativement complexe à fabriquer, puisqu'il met en oeuvre une opération de surmoulage d'éléments distincts réalisés en des matériaux différents.

En outre, il s'avère que la conception de cet anneau, si elle représente un pas important vers l'élimination de plis au niveau de la zone de contact avec l'estomac, n'élimine pas pour autant totalement la probabilité d'occurrence de plis ou discontinuités sur cette zone de contact.

### EXPOSE DE L'INVENTION

L'objet assigné à l'invention vise en conséquence à proposer un nouvel anneau chirurgical, notamment gastrique, permettant de porter remède aux différents inconvénients énumérés précédemment et qui soit particulièrement lisse et atraumatique afin d'être bien supporté par le patient, tout en étant robuste, de conception simplifiée et d'un coût réduit.

Un autre objet de l'invention vise à proposer un nouvel anneau chirurgical, notamment gastrique, dont l'atraumaticité est particulièrement optimisée.

Un autre objet de l'invention vise à proposer un nouvel anneau chirurgical, notamment gastrique, qui permet un réglage fin de la section d'ouverture du stoma.

Un autre objet de l'invention vise à proposer un nouvel anneau chirurgical, notamment gastrique, dont la fabrication met en oeuvre un minimum de composants différents.

Un autre objet de l'invention vise à proposer un nouvel anneau chirurgical, notamment gastrique, qui présente une grande simplicité de fabrication, tout en étant particulièrement compact et léger.

Un autre objet de l'invention vise à proposer un nouvel anneau chirurgical, notamment gastrique, qui présente un excellent compromis entre atrumatisme et simplicité de conception et de fabrication.

L'objet assigné à l'invention vise également à proposer un nouveau procédé de fabrication d'un anneau chirurgical, notamment gastrique, ledit procédé étant particulièrement simplifié et rapide, tout en permettant d'obtenir un anneau chirurgical robuste et atraumatique.

Un autre objet de l'invention vise à proposer un nouveau procédé de fabrication d'un anneau chirurgical, notamment gastrique, particulièrement économique et facile à mettre en oeuvre.

Un autre objet de l'invention vise à proposer un nouveau procédé de fabrication d'un anneau chirurgical, notamment gastrique, permettant d'obtenir un anneau dont l'atraumaticité est particulièrement optimisée.

Un autre objet de l'invention vise à proposer un nouveau procédé de fabrication d'un anneau chirurgical, notamment gastrique, qui permette d'obtenir un anneau permettant un réglage fin de la section d'ouverture du stoma.

Un autre objet de l'invention vise à proposer un nouveau procédé de fabrication d'un anneau chirurgical, notamment gastrique, permettant de réduire le nombre d'étapes de fabrication.

Les objets assignés à l'invention sont atteints à l'aide d'un anneau chirurgical destiné à être implanté dans le corps d'un patient autour d'organe(s) biologique(s) constituant une poche ou un conduit pour modifier la section de passage dudit organe lorsqu'il est enserré par l'anneau, ledit anneau étant formé par une bande souple destinée à être fermée sensiblement vers ses deux extrémités pour former une boucle close, ladite bande comportant une chambre de compression annulaire destinée à contenir un fluide de remplissage, ladite chambre étant délimitée d'une part par une paroi interne destinée à être en contact avec l'organe à enserrer, et d'autre part par une paroi dorsale, caractérisé en ce que ladite paroi dorsale est formée d'un boudin présentant une face intérieure située en regard de la chambre, ladite face intérieure étant pourvue d'au moins une entaille longitudinale pour influer sur la déformation de la paroi interne en vue de limiter la présence d'irrégularités de surface au niveau de la paroi interne lorsque la bande forme une boucle close.

Les objets assignés à l'invention sont également atteints à l'aide d'un procédé de fabrication d'un anneau chirurgical destiné à être implanté dans le corps d'un patient autour d'organe(s) biologique(s) constituant une poche ou un conduit pour modifier la section de passage dudit organe lorsqu'il est enserré par l'anneau, dans lequel on réalise une chambre de compression annulaire destinée à contenir un fluide de remplissage, ladite chambre étant délimitée d'une part par une paroi interne destinée à être en contact avec l'organe à enserrer et d'autre part par une paroi dorsale, ledit procédé étant caractérisé en ce qu'il comprend une étape de réalisation d'un boudin destiné à former la paroi dorsale, ledit boudin présentant une face intérieure destinée à se trouver en regard de la chambre, ainsi qu'une étape de réalisation sur ladite face intérieure d'au moins une entaille longitudinale pour influer sur la déformation de la paroi interne en vue de limiter la présence d'irrégularités de surface au niveau de la paroi interne lorsque la bande forme une boucle close.

### DESCRIPTIF SOMMAIRE DES DESSINS

D'autres objets et avantages de l'invention apparaîtront mieux à la lecture de la description qui suit, ainsi qu'à l'aide des dessins annexés, donnés à titre purement illustratif et non limitatif, dans lesquels :
- La figure 1 illustre, selon une vue latérale en perspective, un anneau de gastroplastie conforme à l'invention dans sa position desserrée.
- La figure 2 illustre, selon une vue identique à celle de la figure 1, un anneau de gastroplastie conforme à l'invention dans sa position fermée.
- La figure 3 illustre, selon une vue en coupe transversale, une première variante de réalisation d'un anneau de gastroplastie conforme à l'invention.
- La figure 4 illustre, selon une vue en coupe transversale, une deuxième variante de réalisation d'un anneau de gastroplastie conforme à l'invention.

### MEILLEURE MANIERE DE REALISER L'INVENTION

Dans la description qui suit, il sera fait référence, uniquement à titre d'exemple et à des fins de simplicité de description, à un anneau de gastroplastie (ou anneau gastrique) conçu pour être implanté autour de l'estomac pour réduire le diamètre de l'ouverture du stoma, ou conçu pour être implanté autour de l'oesophage.

Cependant, l'invention n'est nullement limitée à cette application, et vise au contraire à couvrir également d'autres anneaux chirurgicaux, et de manière générale les anneaux chirurgicaux destinés à être implantés dans le corps d'un patient autour d'organe(s) biologique(s) constituant une poche ou un conduit, pour modifier la section de passage dudit organe lorsqu'il est enserré par l'anneau.

A titre d'exemple, on peut citer les anneaux utilisés pour traiter l'incontinence urinaire ou fécale, ou encore ceux utilisés autour de vaisseaux sanguins pour régler le débit sanguin. Dans le cas de traitement d'incontinence urinaire, l'anneau sera implanté autour de la vessie ou des voies urinaires, et dans le cas de traitement d'incontinence fécale, il sera implanté autour-des voies gastro-intestinales et notamment autour des structures anales de l'intestin.

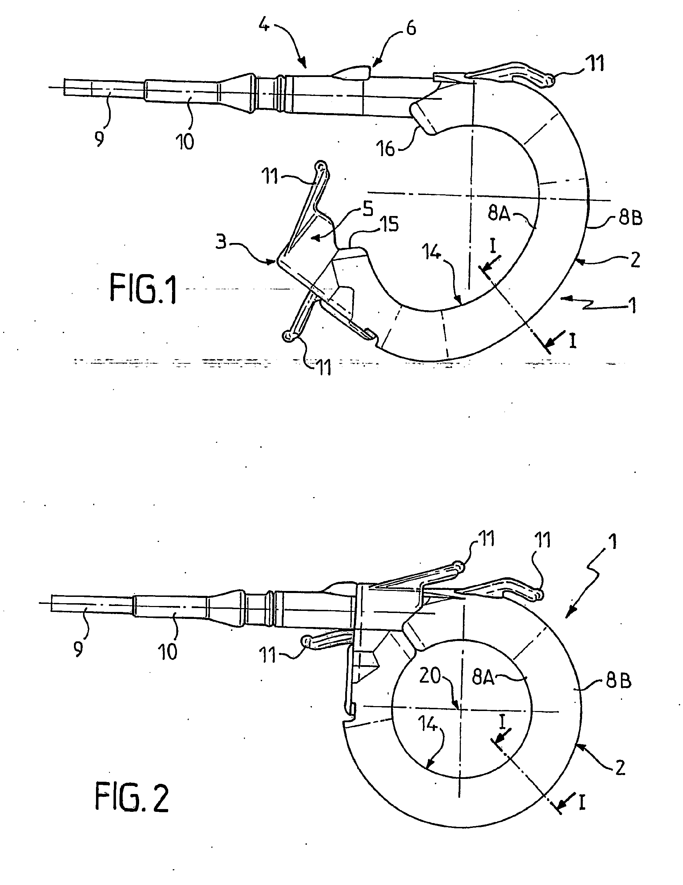

Les figures 1 à 4 illustrent un anneau de gastroplastie 1 conforme à l'invention formé par une bande souple 2 réalisée de préférence à partir d'un matériau élastomère, par exemple du silicone, destinée à être fermée sensiblement vers ses deux extrémités 3, 4 par des moyens de fermeture 5, 6 autour de l'estomac d'un patient, pour former une boucle close, en vue de réduire le diamètre de l'ouverture du stoma.

La position de fermeture de l'anneau est illustrée à la figure 2, position dans laquelle les moyens de fermeture 5, 6 coopèrent entre eux pour assurer le verrouillage de l'anneau 1.

De manière préférentielle, l'anneau chirurgical conforme à l'invention présente une mémoire de forme sensiblement circulaire, de manière à faciliter le positionnement de l'anneau par le chirurgien, puisque dans sa position de repos lâche ouverte (figure 1) l'anneau présente déjà une forme quasi ou sensiblement circulaire proche de sa configuration finale illustrée à la figure 2.

Avantageusement, l'anneau de gastroplastie 1 conforme à l'invention peut être pourvu d'une ou plusieurs languettes 11 de préhension disposées à des endroits prédéterminés, par exemple vers les extrémités 3, 4, de manière à faciliter la manipulation de l'anneau, et en particulier sa fermeture, et surtout son ouverture ou déverrouillage.

Conformément à l'invention, la bande souple 2 comporte une chambre de compression 7 s'étendant longitudinalement sur la majeure partie de la longueur de la bande souple 2. Les moyens de fermeture 5, 6 étant solidaires des extrémités 3, 4 de la bande souple 2 et s'étendant vers l'extérieur de ladite bande 2, la chambre de compression 7 se terminera avantageusement par deux sections transversales 15, 16 sensiblement planes, pour venir en appui l'une contre l'autre dans la position de fermeture de l'anneau (figure 2), de façon à former une chambre de compression annulaire 7 susceptible d'enserrer l'estomac sur une plage angulaire égale ou sensiblement égale à 360 degrés.

La chambre de compression annulaire 7 est destinée à contenir un fluide de remplissage, pour conférer à l'anneau ses dimensions fonctionnelles lui permettant, lorsqu'il encercle un organe tel que l'estomac, de réduire la section de passage de l'estomac à une dimension prédéterminée, fonction de la quantité de fluide présent dans la chambre. En d'autres termes, la chambre 7 est destinée à être « *gonflée* » par le fluide remplissage afin que l'anneau présente une surface de contact avec l'estomac (ou un autre organe dans le cas d'une application autre que la gastroplastie) relativement souple et de diamètre prédéterminé.

De manière préférentielle et connue en soi, la chambre de compression annulaire 7 est à volume réglable, c'est-à-dire que son expansion diamétrale peut être réglée en expansion ou en rétraction, de manière à régler concomitamment le diamètre de l'ouverture du stoma. Plus particulièrement, la chambre 7 est une chambre à volume réglable par injection ou retrait de fluide de remplissage. A cette fin, la chambre de compression annulaire 7 est de préférence reliée par l'ouverture 8 et par un cathéter 9 associé à l'ouverture 8, à un dispositif de réglage (non représenté aux figures) du -diamètre de ladite chambre 7 par injection ou retrait de fluide. De manière connue, le dispositif de réglage est formé par un boîtier miniaturisé qui peut être implanté sous la peau du patient, le boîtier comportant une membrane auto-obturante destinée à être percée par une seringue permettant d'injecter ou de retirer une certaine quantité de fluide (en général de l'eau physiologique) servant à assurer la variation de volume de la chambre de compression annulaire 7.Tel qu'illustré aux figures 1 et 2, le cathéter 9 peut être relié à la bande souple 2 par l'intermédiaire d'un organe de connexion, tel qu'un embout 10.

Il est bien entendu tout à fait envisageable, sans pour autant sortir du cadre de l'invention, que la chambre 7 présente un volume prédéterminée fixe et non réglable, obtenu par exemple au moyen d'un remplissage unique et définitif, lors de la fabrication de l'anneau, de la chambre 7 par une quantité prédéterminée de fluide de remplissage, ladite chambre 7 étant ensuite obturée définitivement, de sorte que toute opération d'injection ou de retrait de fluide est exclue lors de la mise en oeuvre de l'anneau.

Conformément à l'invention, la chambre 7 est délimitée longitudinalement d'une part par une paroi interne 8A, et d'autre part par une paroi dorsale 8B opposée.

La paroi interne 8A est avantageusement formée d'une membrane, c'est-à-dire d'une cloison mince et souple, de préférence pleine, présentant un caractère élastique.

La paroi interne 8A est avantageusement destinée à entrer en contact, ou à être en contact, avec l'organe biologique, en l'occurrence l'estomac, à enserrer. La paroi interne 8A est à ce titre située de préférence sur le périmètre interne 14 de la bande souple 2.

La paroi interne 8A est réalisée à partir d'un premier matériau élastomère, du genre silicone, qui présente de préférence un caractère lisse et biocompatible, de manière à être facilement supporté par les tissus de l'estomac. De manière préférentielle, ce premier matériau élastomère présente une dureté comprise entre 25 et 45 Shore A, et de manière encore plus préférentielle une dureté de l'ordre de 30 Shore A.

Selon une caractéristique importante de l'invention, la paroi dorsale 8B est quant à elle formée d'un boudin, c'est-à-dire d'un bourrelet allongé, de préférence plein et massif, dont la section transversale, contrairement à celle d'une membrane, s'étend de manière sensiblement équivalente, ou du moins comparable, selon deux directions de l'espacé.

En d'autres termes, la paroi dorsale 8B présente en moyenne une épaisseur qui est significativement supérieure à celle de la paroi interne 8A, et par exemple pouvant atteindre, à son maximum, 5 à 10 fois l'épaisseur de ladite paroi interne 8A.

Ce boudin formant paroi dorsale 8B est positionnée à l'extérieur de la bande souple 2, c'est-à-dire sur le périmètre externe de l'anneau lorsque ce dernier occupe sa position de fermeture, tel qu'illustré à la figure 2.

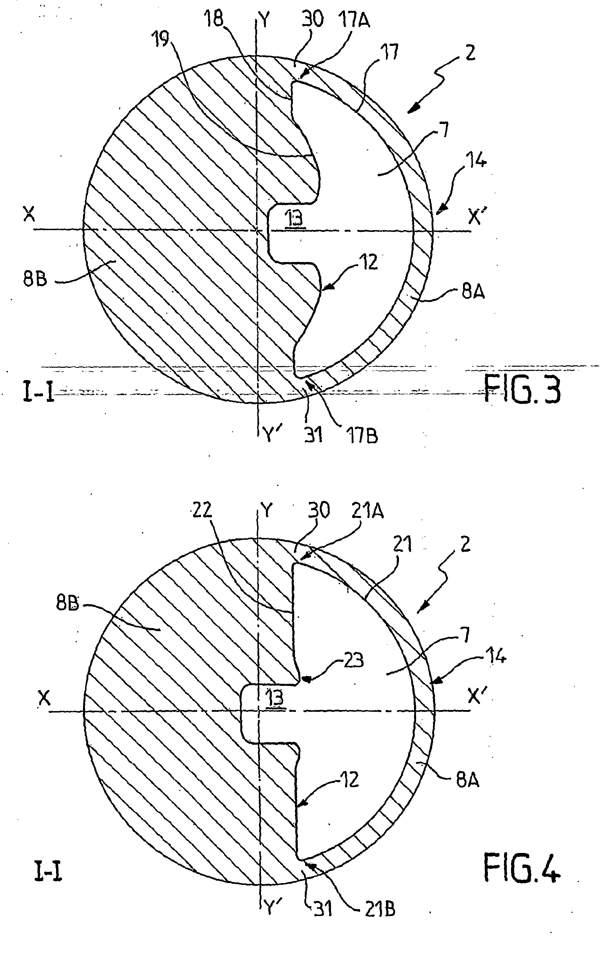

Tel que cela est illustré aux figures 3 et 4, les parois interne 8A et dorsale 8B sont raccordées au niveau d'un point de jonction supérieur 30 et d'un point de jonction inférieur 31, de façon à délimiter latéralement la chambre 7.

Le boudin formant paroi dorsale 8B présente par ailleurs une face intérieure 12 située en regard de la chambre 7, c'est-à-dire formant l'interface entre la paroi dorsale 8B et la chambre 7.

Ladite face intérieure 12 est pourvue d'au moins une entaille longitudinale 13 pour influer sur la déformation de la paroi interne 8A en vue de limiter la présence d'irrégularités de surface au niveau de la paroi interne 8A lorsque la bande 2 forme une boucle close.

En d'autres termes, une entaille ou encoche 13 est ménagée sur la face intérieure 12, ladite entaille 13 s'étendant de préférence sensiblement sur toute la longueur du boudin formant la paroi dorsale 8B, à la manière d'un sillon.

L'entaille longitudinale 13 débouche dans la chambre 7, c'est-à-dire qu'elle forme une rainure à la surface de la face intérieure 12, ladite rainure ménageant ainsi un creux dans la face 12, creux qui communique directement avec la chambre 7.

Ainsi que cela a dejà évoqué précédemment, l'entaille longitudinale (ou les entailles) 13 est pratiquée dans la face intérieure 12 pour générer une modification du comportement mécanique de la paroi interne 8A conduisant à éviter en particulier le plissage de ladite paroi interne 8A lorsque la bande souple 2 est fermée.

En particulier, dans le cas d'une chambre 7 à volume réglable, il a été constaté que la présence d'une ou de plusieurs entaille(s) 13 permettait « *d'accommoder* » les variations d'aire de la paroi interne 8A engendrées par les variations de volume de la chambre 7, et d'éviter ainsi sensiblement la formation de plis ou d'irrégularités qui pourraient apparaître en conséquence de ces variations d'aire.

Dans ce qui suit, on fera référence à un boudin dont la face intérieure 12 est pourvue d'une seule et unique entaille longitudinale 13, étant entendu qu'il est tout à fait envisageable, sans pour autant sortir du cadre de l'invention, que la face-intérieure 12 soit pourvue de plusieurs entailles, éventuellement de profils, de directions et de dimensions différentes.

L'homme du métier, sur la base de l'enseignement de l'invention, pourra ainsi positionner, conformer et dimensionner l'entaille (ou les entailles) 13 pour obtenir et optimiser l'effet recherché d' « *anti-plicature* » de la paroi interne 8A, laquelle conservera ainsi en permanence un caractère sensiblement lisse.

Le principe général de l'invention repose donc sur l'association d'une paroi déformable destinée à venir en contact avec l'estomac et d'un boudin de caractère plus rigide pourvu d'au moins une entaille, lesdits boudin entaillé et membrane délimitant une chambre destinée à accueillir un fluide de gonflage, l'apport dudit fluide de gonflage provoquant la déformation de la membrane dans la direction centripète.

Il a été constaté par la demanderesse que l'association des composants précités conduisait à l'obtention d'une bande souple 2 dont la compression est majoritairement sinon exclusivement centripète, c'est-à-dire dirigée sensiblement vers le centre théorique 20 de l'anneau, tout en présentant au niveau du diamètre intérieur 14 de l'anneau 1, destiné à entrer en contact avec l'estomac, un état de surface particulièrement lisse et régulier, sensiblement exempt de pli, irrégularité ou discontinuité.

L'invention permet donc l'obtention d'une zone interne 14 de l'anneau sensiblement lisse et sans pli quel que soit le niveau de remplissage (et donc de compression) de la chambre 7, cet effet étant obtenu grâce à la présence de l'entaille 13. Au passage, on peut noter que l'on pourrait peut être associer à l'entaille 13 une fonction d'augmentation du moment d'inertie de la section transversale du boudin 8B.

Avantageusement, le boudin formant la paroi dorsale 8B présente un caractère homogène, c'est à dire notamment qu'il est exempt d'irrégularité de matière ou de propriété. De préférence, l'entaille longitudinale 13 est ménagée intégralement dans'le boudin, et est à ce titre exclusivement délimitée par la matière homogène formant le boudin.

Avantageusement, la paroi dorsale 8B est réalisée à partir d'un deuxième matériau élastomère, par exemple du silicone.

De préférence, ledit deuxième matériau est identique, c'est-à-dire similaire en tous points (nature, composition, propriétés) au premier matériau à partir duquel est réalisée la paroi interne 8A.

De préférence, lesdits premier et deuxième matériaux présentent des duretés sensiblement identiques, ou du moins comparables.

Ainsi, contrairement aux dispositifs de l'art antérieur, l'invention peut permettre d'obtenir un excellent effet technique d'atraumatisme, supérieur à celui obtenu dans l'art antérieur, tout en ne mettant en oeuvre, pour la réalisation de la chambre, qu'un seul et unique matériau élastomère, au lieu de deux matériaux de duretés différentes, comme dans l'art antérieur.

Cela présente bien sûr un intérêt considérable d'un point de vue industriel et économique, puisque la mise en oeuvre d'un matériau unique contribue à faciliter les opérations de fabrication et donc à diminuer le prix de revient de l'anneau.

Il est cependant tout à fait envisageable que les parois dorsale 8B et interne 8A soient réalisées à partir de matériaux différents, sans que l'on sorte du cadre de la présente invention.

Avantageusement, la paroi interne 8A vient de matière avec la paroi dorsale 8B, c'est-à-dire que les parois interne 8A et dorsale 8B forment dès leur origine, au moment de leur genèse, un ensemble unitaire monobloc et homogène, sans qu'il y ait eu nécessité d'une opération d'assemblage (collage, surmoulage) entre elles.

On obtient ainsi directement une bande souple 2 particulièrement compacte et robuste, à moindre coût.

Avantageusement, la face intérieure 12 du boudin formant la paroi dorsale 8B est pourvue d'une entaille longitudinale 13 unique positionnée sensiblement au centre de ladite face 12, tel que cela est illustré aux figures 3 et 4.

De façon préférentielle, l'entaille longitudinale 13 présente une section transversale de forme rectangulaire (entaille en U), tel que cela est illustré aux figures 3 et 4. Il est cependant tout à fait envisageable que l'entaille longitudinale adopte un autre profil de section, par exemple triangulaire (entaille en V), sans pour autant que l'on sorte du cadre de l'invention.

On va maintenant décrire plus particulièrement les deux modes de réalisation représentés respectivement aux figures 3 et 4.

Dans ces deux modes de réalisation, la bande souple 2 se présente sous la forme d'un tube plein dé section transversale dont le contour est sensiblement elliptique. Le petit axe de symétrie X-X' de ladite ellipse s'étend sensiblement dans la direction radiale définie par l'anneau 1 fermé de centre 20, tandis que le grand axe Y-Y' s'étend, de façon classique perpendiculairement au petit axe X-X'.

Cette forme elliptique permet de conférer à la bande 2 une surface d'appui 14 relativement large, en tout cas supérieure à celle des chambres annulaires classiques de section circulaire. Cette surface d'appui relativement large, ou en tout cas augmentée, permet de réduire la pression de contact entre l'estomac et l'anneau, du fait de l'augmentation relative de la surface de contact, ce qui réduit encore l'agression sur les tissus de l'estomac.

Avantageusement, la section transversale elliptique de la bande 2 sera sensiblement constante sur toute la longueur développée de ladite bande 2.

Le tube plein formant la bande 2 est évidé longitudinalement, de façon à former à la fois la chambre 7 et l'entaille longitudinale 13. Lesdites chambre 7 et entaille 13 communiquent pour former une cavité unique 7, 13 qui présente une forme s'apparentant, en section transversale, sensiblement à celle d'un champignon dont le pied est formé par l'entaille 13 tandis que le chapeau est formé par la chambre 7.

On va maintenant décrire plus en détails la première variante de réalisation illustrée à la figure 3.

Dans cette variante, la section transversale de la chambre 7, qui forme le chapeau du champignon évoqué précédemment, présente sensiblement une allure générale de croissant, c'est-à-dire, qu'à la manière d'un croissant de lune (ou de disque), ladite section transversale de la chambre 7 est délimitée par deux lignes courbes, incurvées dans le même sens, et jointes à leurs extrémités.

Ainsi, la section transversale de la chambre 7 représentée à la figure 3 est délimitée par une première ligne courbe 17, qui peut être assimilée sensiblement à une portion d'ellipse, ellipse dont le petit axe de symétrie est confondu avec le petit axe X-X' de l'ellipse formant le contour de la bande souple 2.

La première ligne courbe 17 s'étend entre une première extrémité 17A et une deuxième extrémité 17B.

Cette première ligne courbe 17 est reliée, au niveau de sa première extrémité 17A, à un premier tronçon rectiligne 18 qui s'étend sensiblement parallèlement au grand axe Y-Y' de l'ellipse formant le contour de la bande souple 2. Ce premier tronçon rectiligne est lui-même prolongé par une deuxième ligne courbe 19, incurvée vers l'intérieur de la chambre 7, et qui vient sensiblement tangenter le rebord de l'entaille 13.

Des dispositions similaires sont prévues à partir de la deuxième extrémité 17B de la première ligne courbe 17, de telle sorte que l'ensemble formé par la chambre 7 et l'entaille 13 présente une symétrie relativement au petit axe X-X'.

Tel que cela est représenté à la figure 3, l'ensemble formé par la chambre 7 et l'entaille 13 est positionné, en section transversale, entre le grand axe Y-Y' et la paroi interne 8A.

A titre d'exemple, on pourra adopter le dimensionnement suivant pour les différents constituants de l'anneau représenté à la figure 3 :
- épaisseur de la paroi interne 8A égale à 1,1 mm ;
- dimension de l'ellipse formant le contour de la bande souple 2, selon l'axe X-X' égale à 9,5 mm ;
- dimension de l'ellipse formant le contour de la bande souple 2 selon l'axe Y-Y' égale à 12 mm ;
- largeur de l'entaille 13 dans la direction Y-Y' égale à 1,2 mm ;
- distance entre le sommet de la première ligne courbe 17 et le fond
- de l'entaille 13 égale à 3,75 mm ;
- profondeur de l'entaille égale à 1,4 mm.

Il est bien entendu que les différentes valeurs indiquées ci-dessus ne limitent en aucune manière le champ de l'invention, et que ces valeurs pourront en particulier être adaptées par l'homme du métier dans le cadre de ses opérations de conception.

On va maintenant décrire en détails la deuxième variante de réalisation illustrée à la figure 4.

Dans cette variante, la section transversale de la chambre 7, qui forme le chapeau du champignon évoqué précédemment, présente sensiblement une allure générale de quartier, c'est-à-dire qu'à la manière d'un quartier de lune (ou de disque), ladite section transversale de la chambre 7 est délimitée par une ligne courbe incurvée dont les extrémités sont reliées par une ligne sensiblement droite.

Ainsi, la section transversale de la chambre 7 représentée à la figure 4 est délimitée par un premier segment courbe 21, qui peut être sensiblement assimilé à une portion d'ellipse, ellipse dont le petit axe de symétrie est confondu avec le petit axe X-X' de l'ellipse formant le contour de la bande souple 2.

Le premier segment courbe 21 s'étend entre une première extrémité 21A et une deuxième extrémité 21 B. Ce premier segment courbe 21 est reliée, au niveau de sa première extrémité 21A, à un segment rectiligne 22 qui s'étend sensiblement parallèlement au grand axe Y-Y' de l'ellipse formant le contour de la bande souple 2. Ce segment rectiligne 22 vient lui-même accoster le rebord de l'entaille 13, via un deuxième segment courbe 23 incurvé vers la chambre 7, lequel deuxième segment courbe 23 opère un raccordement courbe avec le rebord de l'entaille 13. Le deuxième segment courbe 23 réalise ainsi une légère protubérance au niveau du rebord de l'entaille 13.

Des dispositions similaires sont prévues à partir de la deuxième extrémité 21B du deuxième segment courbe 21, de telle sorte que l'ensemble formé par la chambre 7 et l'entaille 13 présente une symétrie relativement au petit axe X-X'.

Tel que cela est illustré à la figure 4, l'entaille 13 pourra être aménagée de part et d'autre du grand axe Y-Y', dans la direction X-X'.

A titre d'exemple, on pourra adopter le dimensionnement suivant pour les différents constituants de l'anneau représenté à la figure 4 :
- épaisseur de la paroi interne 8A égale à 0,6 mm ;
- dimension de l'ellipse formant le contour de la bande souple 2, selon l'axe X-X' égale à 11,5 mm ;
- dimension de l'ellipse formant le contour de la bande souple 2 selon l'axe Y-Y' égale à 12 mm ;
- largeur de l'entaille 13 dans la direction Y-Y' égale à 2,2 mm ;
- distance entre le sommet du premier segment courbe 21 et le fond de l'entaille 13 égale à 6,35 mm ;
- profondeur de l'entaille égale à 2 mm.
   Il est bien entendu que les différentes valeurs indiquées ci-dessus ne limitent en aucune manière le champ de l'invention, et que ces valeurs pourront en particulier être adaptées par l'homme du métier dans le cadre de ses opérations de conception.

De façon préférentielle, et quel que soit le mode de réalisation adopté, la section transversale de l'entaille 13, tout comme celle de la chambre 7, sera -sensiblement constante sur-toute la longueur développée de la bande 2.

L'invention concerne également un procédé de fabrication d'un anneau chirurgical 1 conforme à l'invention, et en particulier d'un anneau de gastroplastie destiné à être implanté autour de l'estomac ou de l'oesophage.

Selon l'invention, on réalise, lors de la mise en oeuvre de ce procédé, une chambre de compression annulaire 7 destinée à contenir un fluide de remplissage, ladite chambre étant de préférence une chambre à volume réglable par injection ou retrait de fluide de remplissage, ladite chambre 7 étant délimitée d'une part par une paroi interne 8A, de préférence formée d'une membrane, et d'autre part par une paroi dorsale 8B.

Le procédé de fabrication conforme à l'invention comprend une étape de réalisation d'un boudin destiné à former la paroi dorsale 8B, ledit boudin présentant une face intérieure 12 destinée à se trouver en regard de la chambre 7.

Selon une autre caractéristique importante du procédé conforme à l'invention, ce dernier comprend également une étape de réalisation d'au moins une entaille longitudinale 13 sur ladite face intérieure 12 du boudin pour influer sur la déformation de la paroi interne 8A en vue de limiter la présence d'irrégularités de surface au niveau de la paroi interne 8A lorsque la bande 2 forme une boucle close. La fonction de l'entaille 13 a déjà été décrite dans ce qui précède, et il n'est pas nécessaire de la décrire à nouveau en détails ici.

Avantageusement, la chambre 7, les parois interne 8A et dorsale 8B ainsi que ladite au moins une entaille 13 sont réalisées par une opération unique d'injection d'un matériau élastomère dans un moule pourvu d'au moins une empreinte comportant elle-même au moins un noyau.

Ainsi, le procédé conforme à l'invention permet avantageusement d'obtenir la chambre 7, les parois interne 8A et dorsale 8B ainsi que ladite au moins une entaille 13 de manière simultanée et en une seule étape.

De façon encore plus préférentielle, ladite opération unique d'injection est réalisée à l'aide d'un matériau élastomère unique, tel que du silicone de grade biomédical, c'est-à-dire que cette opération d'injection permet d'obtenir en une seule étape la bande souple 2.

Il est cependant tout à fait envisageable que les parois interne 8A et dorsale 8B soient obtenues à l'issue d'opérations d'injection distinctes, mettant en jeu des matériaux différents, et qu'ensuite lesdites parois interne 8A et dorsale 8B soient assemblées, au niveau de leurs points de jonction 30, 31 par tout procédé classique, du genre collage, surmoulage ou autres.

Le procédé tel que décrit précédemment permet ainsi d'obtenir de façon très simple et dans un temps relativement bref une chambre 7 compacte homogène et monobloc.

II est d'ailleurs intéressant de noter que ce procédé, par sa simplicité, peut être facilement automatisé, en faisant intervenir un minimum d'opérations manuelles.

L'invention concerne enfin également une méthode de traitement thérapeutique de l'obésité morbide incluant les étapes d'installation, de contrôlé et de réglage, notamment du diamètre, d'un anneau gastrique conforme à l'invention.

### POSSIBILITE D'APPLICATION INDUSTRIELLE

L'invention trouve son application industrielle dans la conception et la fabrication d'anneaux chirurgicaux destinés à être implantés autour d'organe(s) biologique(s) constituant une poche ou un conduit pour modifier la section de passage dudit organe lorsqu'il est enserré par l'anneau, et en particulier d'anneaux gastriques destinés au traitement de l'obésité.

## Revendications

1. - Anneau chirurgical (1) destiné à être implanté dans le corps d'un patient autour d'organe(s) biologique(s) constituant une poche ou un conduit pour modifier la section de passage dudit organe lorsqu'il est enserré par l'anneau, ledit anneau (1) étant formé par une bande souple (2) destinée à être fermée sensiblement vers ses deux extrémités (3, 4) pour former une boucle close, ladite bande (2) comportant une chambre de compression annulaire (7) destinée à contenir un fluide de remplissage, ladite chambre (7) étant délimitée d'une part par une paroi interne (8A) destinée à être en contact avec l'organe à enserrer, et d'autre part par une paroi dorsale (8B), **caractérisé en ce que** ladite paroi dorsale (8B) est formée d'un boudin présentant une face intérieure (12) située en regard de la chambre (7), ladite face intérieure (12) étant pourvue d'au moins une entaille longitudinale (13) pour influer sur la déformation de la paroi interne (8A) en vue de limiter la présence d'irrégularités de surface au niveau de la paroi interne (8A) lorsque la bande (2) forme une boucle close.

2. - Anneau (1) selon la revendication 1 **caractérisé en ce que** la paroi interne (8A) est formée d'une membrane.

3. - Anneau (1) selon la revendication 1 ou 2 **caractérisé en ce que** la chambre (7) est une chambre à volume réglable par injection ou retrait dudit fluide de remplissage.

4. - Anneau selon l'une des revendications 1 à 3 **caractérisé en ce que** le boudin présente un caractère homogène et **en ce que** l'entaille longitudinale (13) est ménagée intégralement dans le boudin.

5. - Anneau (1) selon l'une des revendications 1 à 4 **caractérisé en ce que** la paroi interne (8A) est réalisée à partir d'un premier matériau élastomère, tandis que la paroi dorsale (8B) est réalisée à partir d'un deuxième matériau élastomère.

6. - Anneau (1) selon la revendication 5 **caractérisé en ce que** lesdits premier et deuxième matériaux sont identiques.

7. - Anneau (1) selon la revendication 6 **caractérisé en ce que** lesdits premier et deuxième matériaux présentent des duretés sensiblement identiques.

8. - Anneau (1) selon l'une revendication 1 à 7 **caractérisé en ce que** la paroi interne (8A) vient de matière avec la paroi dorsale (8B).

9. - Anneau (1) selon l'une des revendications 1 à 8 **caractérisé en ce que** la face intérieure (12) du boudin est pourvue d'une entaille longitudinale (13) unique positionnée sensiblement au centre de ladite face.

10. -Anneau (1) selon l'une des revendications 1 à 9 **caractérisé en ce que** l'entaille longitudinale (13) présente une section transversale de forme sensiblement rectangulaire.

11. -Anneau (1) selon l'une des revendications 1 à 10 **caractérisé en ce que** la bande (2) se présente sous la forme d'un tube plein de section transversale sensiblement elliptique, ledit tube étant évidé de façon à former à la fois la chambre (7) et l'entaille longitudinale (13), lesdites chambre (7) et entaille (13) communiquant pour former une cavité unique (7, 13), dont la forme, en section transversale, s'apparente sensiblement à celle d'un champignon, dont le pied est formé par l'entaille (13), tandis que le chapeau est formé par la chambre (7).

12. -Anneau (1) selon la revendication 11 **caractérisé en ce que** la section transversale de la chambre (7) présente une allure générale de quartier.

13. -Anneau (1) selon la revendication 11 **caractérisé en ce que** la section transversale de la chambre (7) présente une allure générale de croissant.

14. -Anneau (1) selon l'une des revendications 1 à 13 **caractérisé en ce qu'**il constitue un anneau de gastroplastie, destiné à être implanté autour de l'estomac ou de l'oesophage.

15. - Procédé de fabrication d'un anneau chirurgical (1) destiné à être implanté dans le corps d'un patient autour d'organe(s) biologique(s) constituant une poche ou un conduit pour modifier la section de passage dudit organe lorsqu'il est enserré par l'anneau (1), dans lequel on réalise une chambre de compression annulaire (7) destinée à contenir un fluide de remplissage, ladite chambre (7) étant délimitée d'une part par une paroi interne (8A) destinée à être en contact avec l'organe à enserrer et d'autre part par une paroi dorsale (8B), ledit procédé étant **caractérisé en ce qu'**il comprend une étape de réalisation d'un boudin destiné à former la paroi dorsale (8B), ledit boudin présentant une face intérieure (12) destinée à se trouver en regard de la chambre (7), ainsi qu'une étape de réalisation sur ladite face intérieure (12) d'au moins une entaille longitudinale (13) pour influer sur la déformation de la paroi interne (8A) en vue de limiter la présence d'irrégularités de surface au niveau de la paroi interne (8A) lorsque la bande (2) forme une boucle close.

16. -Procédé selon la revendication 15 **caractérisé en ce que** la paroi interne (8A) est formée d'une membrane.

17. -Procédé selon la revendication 15 ou 16 **caractérisé en ce que** la chambre (7) est une chambre à volume réglable par injection ou retrait de fluide de remplissage.

18. -Procédé selon l'une des revendications 15 à 17 **caractérisé en ce que** la chambre (7), les parois interne (8A) et dorsale (8B), ainsi que ladite au moins une entaille (13) sont réalisées par une opération unique d'injection d'un matériau élastomère unique dans un moule.

19. -Procédé selon l'une des revendications 15 à 18 **caractérisé en ce qu'**il constitue un procédé de fabrication d'un anneau (1) de gastroplastie, destiné à être implanté autour de l'estomac ou de l'oesophage.

## Claims

1. Surgical ring (1) intended to be implanted in the body of a patient around biological organ(s) constituting a pouch or a conduit for modifying the cross-section of passage of the said organ when it is clamped by the ring, the said ring (1) being formed by a flexible band (2) intended to be closed substantially towards its two ends (3, 4) in order to form a closed loop, the said band (2) comprising an annular compression chamber (7) intended to contain a filling fluid, the said chamber (7) being delimited firstly by an internal wall (8A) intended to be in contact with the organ to be clamped, and secondly by a dorsal wall (8B), **characterised in that** the said dorsal wall (8B) is formed by a bulge having an internal face (12) situated facing the chamber (7), the said internal face (12) being provided with at least one longitudinal notch (13) for influencing the deformation of the internal wall (8A) with a view to limiting the presence of surface irregularities at the internal wall (8A) when the band (2) forms a closed loop.

2. Ring (1) according to claim 1, **characterised in that** the internal wall (8A) is formed by a membrane.

3. Ring (1) according to claim 1 or 2, **characterised in that** the chamber (7) is a chamber with a volume which is adjustable by injection or withdrawal of the said filling fluid.

4. Ring according to one of claims 1 to 3, **characterised in that** the bulge has a homogeneous character and **in that** the longitudinal notch (13) is formed entirely in the bulge.

5. Ring (1) according to one of claims 1 to 4, **characterised in that** the internal wall (8A) is produced from a first elastomer material, while the dorsal wall (8B) is produced from a second elastomer material.

6. Ring (1) according to claim 5, **characterised in that** the said first and second materials are identical.

7. Ring (1) according to claim 6, **characterised in that** the said first and second materials have substantially identical hardnesses.

8. Ring (1) according to one of claims 1 to 7, **characterised in that** the internal wall (8A) is made in one piece with the dorsal wall (8B).

9. Ring (1) according to one of claims 1 to 8, **characterised in that** the internal face (12) of the bulge is provided with a single longitudinal notch (13) positioned substantially at the centre of the said face.

10. Ring (1) according to one of claims 1 to 9, **characterised in that** the longitudinal notch (13) has a transverse section which is substantially rectangular in shape.

11. Ring (1) according to one of claims 1 to 10, **characterised in that** the band (2) is in the form of a solid tube with a substantially elliptical transverse section, the said tube being hollowed out so as to form both the chamber (7) and the longitudinal notch (13), the said chamber (7) and notch (13) communicating in order to form a single cavity (7, 13), the shape of which, in transverse section, is substantially like that of a mushroom, the foot of which is formed by the notch (13), while the cap is formed by the chamber (7).

12. Ring (1) according to claim 11, **characterised in that** the transverse section of the chamber (7) has the general appearance of a segment.

13. Ring (1) according to claim 11, **characterised in that** the transverse section of the chamber (7) has the general appearance of a crescent.

14. Ring (1) according to one of claims 1 to 13, **characterised in that** it constitutes a gastroplasty ring, intended to be implanted around the stomach or oesophagus.

15. Method of manufacturing a surgical ring (1) intended to be implanted in the body of a patient around biological organ(s) constituting a pouch or a conduit for modifying the cross-section of passage of the said organ when it is clamped by the ring (1), in which an annular compression chamber (7) is produced, intended to contain a filling fluid, the said chamber (7) being delimited firstly by an internal wall (8A) intended to be in contact with the organ to be clamped and secondly by a dorsal wall (8B), the said method being **characterised in that** it comprises a step of producing a bulge which is intended to form the dorsal wall (8B), the said bulge having an internal face (12) intended to be situated facing the chamber (7), and a step of producing on the said internal face (12) at least one longitudinal notch (13) in order to influence the deformation of the internal wall (8A) with a view to limiting the presence of surface irregularities at the internal wall (8A) when the band (2) forms a closed loop.

16. Method according to claim 15, **characterised in that** the internal wall (8A) is formed by a membrane.

17. Method according to claim 15 or 16, **characterised in that** the chamber (7) is a chamber with a volume adjustable by the injection or withdrawal of filling fluid.

18. Method according to one of claims 15 to 17, **characterised in that** the chamber (7), the internal (8A) and dorsal (8B) walls, as well as the said at least one notch (13), are produced by a single operation of injecting a single elastomer material into a mould.

19. Method according to one of claims 15 to 18, **characterised in that** it constitutes a method of manufacturing a gastroplasty ring (1), intended to be implanted around the stomach or oesophagus.

## Patentansprüche

1. - Chirurgischer Ring (1), dazu vorgesehen, im Körper eines Patienten um ein biologisches Organ/biologische Organe implantiert zu werden, umfassend eine Tasche oder einen Kanal, um den Abschnitt des Durchgangs des Organs zu modifizieren, wenn es vom Ring umschlossen ist, wobei der Ring (1) aus einem flexiblen Band (2) gebildet ist, das dazu vorgesehen ist, im Wesentlichen gegen seine zwei Enden (3, 4) geschlossen zu sein, um eine geschlossene Schlinge zu bilden, wobei das Band (2) eine ringförmige Druckkammer (7) umfasst, die dazu vorgesehen ist, eine Füllflüssigkeit zu enthalten, wobei die Kammer (7) einerseits von einer inneren Wand (8A), die dazu vorgesehen ist, mit dem zu umschließenden Organ in Kontakt zu sein, und andererseits von einer Rückwand (8B) begrenzt ist, **dadurch gekennzeichnet, dass** die Rückwand (8B) aus einem Wulst geformt ist, der eine innere Seite (12) aufweist, die sich gegenüber der Kammer (7) befindet, wobei die innere Seite (12) mit mindestens einer Längskerbe (13) ausgestattet ist, um die Verformung der inneren Wand (8A) zu beeinflussen, um die Anwesenheit von Unregelmäßigkeiten der Oberfläche auf der Ebene der inneren Wand (8A) zu begrenzen, wenn das Band (2) eine geschlossene Schlinge bildet.

2. - Ring (1) nach Anspruch 1, **dadurch gekennzeichnet, dass** die innere Wand (8A) aus einer Membran gebildet ist.

3. - Ring (1) nach Anspruch 1 oder 2, **dadurch gekennzeichnet, dass** die Kammer (7) eine Kammer mit einem Volumen, das durch das Einspritzen oder das Entfernen der Füllflüssigkeit regulierbar ist, ist.

4. - Ring nach einem der Ansprüche 1 bis 3, **dadurch gekennzeichnet, dass** der Wulst einen homogenen Charakter aufweist, und dass die Längskerbe (13) vollständig in den Wulst aufgenommen ist.

5. - Ring (1) nach einem der Ansprüche 1 bis 4, **dadurch gekennzeichnet, dass** die innere Wand (8A) aus einem ersten Elastomer-Werkstoff hergestellt ist, während die Rückwand (8B) aus einem zweiten Elastomer-Werkstoff hergestellt ist.

6. - Ring (1) nach Anspruch 5, **dadurch gekennzeichnet, dass** der erste und der zweite Werkstoff identisch sind.

7. - Ring (1) nach Anspruch 6, **dadurch gekennzeichnet, dass** der erste und der zweite Werkstoff im Wesentlichen identische Härten aufweisen.

8. - Ring (1) nach einem der Ansprüche 1 bis 7, **dadurch gekennzeichnet, dass** die innere Wand (8A) einstückig mit der Rückwand (8B) ist.

9. - Ring (1) nach einem der Ansprüche 1 bis 8, **dadurch gekennzeichnet, dass** die innere Seite (12) des Wulstes mit einer einzigen Längskerbe (13), die im Wesentlichen im Zentrum der besagten Seite angebracht ist, versehen ist.

10. - Ring (1) nach einem der Ansprüche 1 bis 9, **dadurch gekennzeichnet**, das die Längskerbe (13) einen im Wesentlichen rechteckigen Querschnitt aufweist.

11. - Ring (1) nach einem der Ansprüche 1 bis 10, **dadurch gekennzeichnet, dass** das Band (2) die Form eines vollen Rohres mit einem im Wesentlichen elliptischen Querschnitt aufweist, wobei das Rohr so ausgehöhlt ist, dass es gleichzeitig die Kammer (7) und die Längskerbe (13) bildet, wobei die Kammer (7) und die Kerbe (13) miteinander verbunden sind, um einen einzigen Hohlraum (7, 13) zu bilden, dessen Form im Querschnitt im Wesentlichen derjenigen eines Pilzes gleicht, dessen Fuß durch die Kerbe (13) gebildet ist, während der Hut durch die Kammer (7) gebildet ist.

12. - Ring (1) nach Anspruch 11, **dadurch gekennzeichnet, dass** der Querschnitt der Kammer (7) das allgemeine Aussehen eines Viertels aufweist.

13. - Ring (1) nach Anspruch 11, **dadurch gekennzeichnet, dass** der Querschnitt der Kammer (7) das allgemeine Aussehen einer Sichel aufweist.

14. - Ring (1) nach einem der Ansprüche 1 bis 13, **dadurch gekennzeichnet, dass** er einen Gastroplastikring, der dazu vorgesehen ist, um den Magen oder die Speiseröhre implantiert zu werden, bildet.

15. - Verfahren zur Herstellung eines chirurgischen Rings (1), dazu vorgesehen, im Körper eines Patienten um ein biologisches Organ/biologische Organe implantiert zu werden, umfassend eine Tasche oder einen Kanal, um den Abschnitt des Durchgangs des Organs zu modifizieren, wenn es vom Ring (1) umschlossen ist, in dem eine ringförmige Druckkammer (7) durchgeführt wird, die dazu vorgesehen ist, eine Füllflüssigkeit zu enthalten, wobei die Kammer (7) einerseits von einer inneren Wand (8A), die dazu vorgesehen ist, mit dem zu umschließenden Organ in Kontakt zu sein, und andererseits von einer Rückwand (8B) begrenzt ist, wobei das Verfahren **dadurch gekennzeichnet ist, dass** es einen Schritt zur Erzeugung eines Wulstes umfasst, der dazu vorgesehen ist, die Rückwand (8B) zu bilden, wobei der Wulst eine innere Seite (12) aufweist, die dazu vorgesehen ist, gegenüber der Kammer (7) angebracht zu sein, ebenso wie ein Schritt zur Erzeugung auf der inneren Seite (12) von mindestens einer Längskerbe (13), um die Verformung der inneren Wand (8A) zu beeinflussen, um die Anwesenheit von Unregelmäßigkeiten der Oberfläche auf der Ebene der inneren Wand (8A) zu begrenzen, wenn das Band (2) eine geschlossene Schlinge bildet.

16. - Verfahren nach Anspruch 15, **dadurch gekennzeichnet, dass** die innere Wand (8A) aus einer Membran gebildet ist.

17. - Verfahren nach Anspruch 15 oder 16, **dadurch gekennzeichnet, dass** die Kammer (7) eine Kammer mit einem Volumen, das durch das Einspritzen oder das Entfernen der Füllflüssigkeit regulierbar ist, ist.

18. - Verfahren nach einem der Ansprüche 15 bis 17, **dadurch gekennzeichnet, dass** die Kammer (7), die innere Wand (8A) und die Rückwand (8B) ebenso wie die mindestens eine Kerbe (13) durch einen einzigen Vorgang des Einspritzens eines einzigen Elastomer-Werkstoffes in eine Form durchgeführt werden.

19. - Verfahren nach einem der Ansprüche 15 bis 18, **dadurch gekennzeichnet, dass** es ein Verfahren zur Herstellung eines Gastroplastikrings (1), der dazu vorgesehen ist, um den Magen oder die Speiseröhre implantiert zu werden, bildet.
